# FASCICULE DE BREVET EUROPEEN

(11) **EP 3 200 710 B1**
(45) Date de publication et mention de la délivrance du brevet: **25.07.2018**
(21) Numéro de dépôt: 15788118.6
(22) Date de dépôt: 30.09.2015
(51) Int. Cl.: A61B 17/70, A61B 17/90, A61B 17/88

(54) **ANCILLAIRE DE MISE EN TENSION D'UN ÉLÉMENT LONGILIGNE**
ZUBEHÖR ZUM SPANNEN EINES LÄNGLICHEN ELEMENTS
ACCESSORY FOR TENSIONING AN ELONGATE ELEMENT

(30) Priorité: 01.10.2014 FR 1459366
(43) Date de publication de la demande: 09.08.2017
(73) Titulaire: Cousin Biotech, 59117 Wervicq Sud (FR)
(72) Inventeur: DENEUVILLERS, Guy, F-62155 Merlimont (FR); PRANDI, Jules, F-59200 Tourcoing (FR)
(74) Mandataire: Balesta, Pierre
(86) Numéro de dépôt international: PCT/FR2015/052617
(87) Numéro de publication internationale: WO 2016/051088

(56) Documents cités:
- EP-A1- 1 878 397
- FR-A1- 2 890 851

## Description

La présente invention concerne le domaine technique des ancillaires de mise en tension d'un élément longiligne, notamment plat, pour la fixation d'un implant sur un élément osseux, notamment au moins une portion d'un corps vertébral.

### Arrière-plan de l'invention

Il est connu de corriger les courbures du rachis anormales et/ou les inclinaisons pathologiques des vertèbres entre-elles par l'utilisation de barres longitudinales disposées de part et d'autre de la colonne vertébrale et solidarisées aux vertèbres par l'intermédiaire de vis insérées dans les vertèbres elles-mêmes ou de crochets, introduits par exemple le long du canal rachidien.

Ces vis et crochets ont cependant pour inconvénients d'être agressifs pour le rachis.

Pour pallier à ces inconvénients, il a été proposé un élément longiligne flexible de fixation combiné avec un implant apte à être solidarisé à une barre longitudinale, l'élément longiligne étant fixé à l'implant et venant en prise avec au moins une portion vertébrale en formant une boucle autour de cette dernière.

Les barres longitudinales peuvent être mises en oeuvre dans le traitement de la scoliose ou encore le traitement de pathologies dégénératives. On rencontre différents niveaux de dégénérescence, par exemple sur un niveau vertébral telle qu'une discopathie ou sur deux niveaux vertébrales tel que le spondylolisthésis ou encore plusieurs niveaux comme la scoliose en C ou en S.

Dans ces traitements, la barre longitudinale peut être mise en liaison avec le niveau vertébral à corriger par l'intermédiaire d'un élément longiligne, lequel passe autour au moins d'une portion d'un corps vertébral et autour de ladite barre puis les extrémités dudit élément longiligne peuvent être maintenues au moyen d'une suture, d'un noeud ou à l'aide d'un moyen de solidarisation implantable. Ledit moyen de solidarisation peut par exemple comprendre un logement recevant la barre longitudinale et l'élément longiligne à l'état enroulé autour de cette dernière, et des moyens de serrage pour le blocage de la barre et dudit élément longiligne dans ledit logement. Généralement, les moyens de serrage comprennent des moyens de pincement dudit élément longiligne empêchant tout mouvement de ce dernier.

L'un des problèmes que cherche à résoudre la présente invention est de mettre en tension l'élément longiligne lorsqu'il forme une boucle sur au moins une portion d'un corps vertébral, et est en prise avec un implant et/ou une barre longitudinale.

EP 1.933.743 B1 a pour objet un ancillaire de mise en tension d'un élément longiligne comprenant une pièce cylindrique mobile apte à se déplacer en translation autour d'une tige, ladite tige comprenant à son extrémité distale des moyens d'appui sur une barre longitudinale et non sur l'implant recevant la barre et l'élément longiligne. La pièce cylindrique mobile comprend également un ergot pour la fixation de l'élément longiligne. Le système de serrage dynamométrique est actionnable par l'intermédiaire d'une poignée. La mise en tension de l'élément longiligne est effectuée par le chirurgien en pressant la poignée sans limite de la tension qui peut être appliquée. Aucune partie de l'ancillaire n'est adaptée pour autoriser le déplacement de l'élément longiligne, ce dernier étant simplement tendu entre deux points (c'est-à-dire entre l'implant et l'ergot) et mis en tension sans qu'il n'y ait un quelconque déplacement de l'élément longiligne dans une partie de l'ancillaire. Avec ce système de serrage, le chirurgien peut appliquer une tension trop importante sur l'élément longiligne, ce qui risque de nuire au réglage de la ou des barres longitudinale(s) placée(s) sur le ou les niveau(x) vertébral(aux) à corriger, voir même de déplacer des vertèbres. En outre, la tension est appliquée sur une boucle dans le prolongement des deux extrémités libres de l'élément longiligne solidarisées à l'aide d'un passant. La tension appliquée ne sera donc pas la même sur chacune des extrémités libres puisqu'elle est appliquée directement sur une seule extrémité libre de l'élément longiligne dans le prolongement de l'autre extrémité libre par l'intermédiaire de la boucle. L'application de la tension est donc déséquilibrée sur chaque extrémité libre de l'élément longiligne et risque d'entraîner l'érosion de la portion vertébrale sur laquelle la tension est appliquée et/ou le glissement de l'élément longiligne sur ladite portion vertébrale.

FR 2.981.841 A1 a pour objet un ancillaire de mise en tension cherchant à éviter comparativement à EP 1.933.743 B1 que l'élément longiligne ne soit vrillé lorsqu'il est mis en tension. L'ancillaire comprend ainsi un renvoi afin que l'élément longiligne forme un angle de 90°. Les extrémités de l'élément longiligne munies de moyens de solidarisation sont fixées à une roue de compensation selon deux points diamétralement opposés. La tension appliquée sur chacune des extrémités de l'élément longiligne n'est donc pas équilibrée. En outre, le renvoi selon un angle de 90° de l'élément longiligne ainsi que le moyen de fixation d'une extrémité dans une saignée engendrent des frottements importants sur l'élément longiligne risquant de le fragiliser. De plus, du fait de l'organe de renvoi, l'ancillaire est encombrant. Or, lors d'une intervention chirurgicale, de multiples implants et barres longitudinales sont placés sur le ou les niveaux vertébral(aux) à corriger de sorte qu'il est important pour le chirurgien une fois un élément longiligne mis en tension de pouvoir accéder facilement à l'implant pour terminer le serrage et la fixation de ce dernier sur l'élément longiligne mais également de pouvoir accéder aux autres implants et/ou barres longitudinales.

### Objet et résumé de l'invention

La présente invention a ainsi pour objet un ancillaire de mise en tension permettant de mettre en tension un élément longiligne formant une boucle sur au moins une portion d'un corps vertébral sans endommager ledit corps vertébral, qui soit progressive et facilement ajustable par le chirurgien.

La présente invention a également pour objet un ancillaire de mise en tension permettant d'exercer une tension égale sur chacune des deux extrémités de l'élément longiligne.

La présente invention a aussi pour objet un ancillaire de mise en tension permettant au chirurgien de relâcher rapidement la tension exercée sur l'élément longiligne si besoin, et d'appliquer une tension sur l'élément longiligne contrôlée, notamment n'excédant pas une valeur déterminée.

La présente invention a pour objet, selon un premier aspect, un ancillaire de mise en tension d'un élément longiligne, notamment plat, pour la fixation d'un implant sur un élément osseux, notamment au moins une portion d'un corps vertébral, en entourant au moins partiellement ledit élément osseux, ledit ancillaire comprenant un corps ayant des extrémités proximale et distale, l'extrémité distale étant munie d'un dispositif pour prendre appui sur l'implant, ledit corps s'étendant selon un axe longitudinal , l'ancillaire comprend en outre un chariot mobile selon l'axe longitudinal et un dispositif de fixation à l'élément longiligne. De manière caractéristique, l'ancillaire comprend un arbre rotatif autour de l'axe longitudinal du corps, cet arbre est configuré pour coopérer avec le chariot de sorte qu'une rotation de l'arbre entraine le déplacement en translation du chariot le long de l'axe longitudinal et corrélativement l'éloignement du chariot de l'extrémité distale du corps et la mise en tension de l'élément longiligne.

Avantageusement, l'ancillaire selon l'invention comprend également un dispositif d'embrayage et de débrayage du chariot de l'arbre rotatif, le chariot ayant une position débrayée dans laquelle le chariot est libre de glisser longitudinalement par rapport à l'axe longitudinal (l) de l'arbre rotatif et une position embrayée dans laquelle le chariot est couplé avec l'arbre rotatif.

Avantageusement, l'élément longiligne est mis en tension selon l'axe longitudinale du corps recevant le chariot mobile ce qui diminue l'encombrement de l'ancillaire en fonctionnement et facilite sa mise en tension.

En particulier, l'élément longiligne est mis tension selon une trajectoire parallèle à l'axe longitudinal s'étendant entre le dispositif d'appui et le dispositif de fixation.

De préférence, le chariot comprend le dispositif de fixation de l'élément longiligne.

De plus, le chariot étant monté selon un arbre rotatif, l'ancillaire selon l'invention n'a pas besoin d'un système anti-retour de l'élément longiligne. En effet, lorsque la tension appliquée sur l'élément longiligne est relâchée, le chariot ne se déplace plus et reste immobile car il est retenu par l'arbre rotatif.

L'élément longiligne selon l'invention est de préférence un élément textile, qui peut être tressé, tricoté ou tissé, de préférence tressé. Encore de préférence, l'élément longiligne est plat afin de générer des forces de frottement importantes avec les surfaces contre lesquelles il se déplace, améliorant ainsi les forces de serrage.

L'élément longiligne comprend des première et seconde extrémités, éventuellement libre(s).

Le dispositif d'appui vient en appui sur l'implant qui peut comprendre un connecteur et/ou un moyen de solidarisation implantable de l'élément longiligne à une barre longitudinale et/ou un ancillaire de mise en pré-tension d'un élément longiligne, tel que celui décrit ci-après en référence à un second aspect selon l'invention.

Le dispositif d'appui de l'ancillaire de mise en tension peut être le dispositif d'appui de l'ancillaire de mise en pré-tension décrit ci-après, indépendamment des autres caractéristiques de l'ancillaire de mise en pré-tension.

Le connecteur peut être celui décrit dans la demande de brevet internationale PCT/FR2014/051801 déposée au nom de la demanderesse, et dont la description est incorporée dans le présent texte.

Le moyen de solidarisation implantable peut être celui décrit dans les brevets EP 2.555.698 B1 et EP 2.609.882 A1.

De préférence, le chariot est configuré pour recevoir au moins deux portions d'élément longiligne superposées ce qui améliore la distribution de la tension dans l'élément longiligne et évite les risques d'érosion et/ou de glissement de l'élément longiligne autour de la portion du corps vertébral.

De préférence, l'arbre rotatif est un arbre fileté, encore de préférence l'arbre comprend un filetage hélicoïdal.

L'expression « apte à » dans le présent texte est équivalente aux expressions suivantes, considérées indépendamment les unes des autres : « destiné à », « configuré pour », « adapté à autoriser », « adapter à se conformer à ».

Dans une variante, l'arbre comporte une portion s'étendant au-delà de l'extrémité proximale du corps configurée pour être couplée avec un dispositif d'actionnement dynamométrique.

Avantageusement, il est possible de choisir précisément le couple (newtons/cm) que l'on souhaite appliquer pour la mise en tension de l'élément longiligne au lieu d'exercer une tension approximative dont on ne connait pas le couple ce qui est plus sûr pour le patient.

Dans une variante, le dispositif de fixation comporte un ensemble formant mâchoires, s'étendant dans deux plans inclinés (p1,p2) l'un par rapport à l'autre et inclinés par rapport à l'axe longitudinal du corps.

De préférence, l'ensemble formant mâchoires, comprend une mâchoire latérale gauche et une mâchoire latérale droite qui s'écartent l'une de l'autre, pour recevoir au moins une portion de l'élément longiligne, chacune dans leur plan incliné respectif (p1,p2).

Selon une autre variante, le dispositif de fixation comporte un ensemble formant mâchoires ayant une mâchoire supérieure et une mâchoire inférieure, la mâchoire supérieure s'étendant dans un plan incliné (p3) par rapport à la mâchoire inférieure et/ou par rapport à l'axe longitudinal du corps cylindrique. De préférence, la mâchoire inférieure est dans un plan (i) parallèle au plan longitudinal (P) du corps cylindrique. De préférence, la mâchoire supérieure s'écarte de la mâchoire inférieure (qui est quant à elle fixe par rapport à la mâchoire supérieure, car solidarisée au dispositif d'embrayage et de débrayage), pour recevoir au moins une portion de l'élément longiligne, dans un plan (T1) transversal, de préférence sensiblement perpendiculaire, au plan longitudinal (P) du corps cylindrique.

Avantageusement, l'élément longiligne (au moins deux portions de ce dernier superposées) est pincé entre lesdites mâchoires supérieure et inférieure en sorte d'être disposé dans un plan parallèle au plan longitudinal (P) du corps cylindrique.

De préférence, selon l'une ou l'autre des deux variantes précédentes, les mâchoires latérales gauche et droite ou les mâchoires supérieure et inférieure, comprennent chacune une face ayant une portion de contact venant en contact avec une portion de l'élément longiligne. Lesdites portions de contact sont moletées, en particulier comprennent un moletage de forme pyramidale. Encore de préférence, le moletage de forme pyramidale d'une portion de contact donnée est disposé en quinconce par rapport au moletage de la portion de contact opposée (disposée en regard de la portion de contact donnée) afin d'améliorer le pincement et donc le blocage de l'élément longiligne.

Selon les deux variantes précédentes, cet ensemble formant mâchoires constitue un dispositif de fixation de l'élément longiligne au chariot.

Le chirurgien n'a pas ainsi besoin de disposer des moyens particuliers sur les extrémités libres de l'élément longiligne pour les faire coopérer avec un système de fixation sur l'ancillaire ou de former une boucle comme cela est le cas dans l'état de la technique. Il suffit ainsi simplement de superposer la première et/ou la seconde extrémité(s) libre(s) de l'élément longiligne dans l'ensemble formant mâchoires après avoir écarté les mâchoires l'une de l'autre à l'aide d'un organe de préhension décrit ci-après.

De préférence, l'inclinaison de l'ensemble formant mâchoires ou de la mâchoire supérieure permet d'appliquer une tension progressive sur l'élément longiligne ce qui améliore la force de fixation de l'élément longiligne au dispositif de fixation. Encore de préférence, les deux plans des mâchoires latérales gauche et droite convergent vers l'extrémité distale du corps, ce qui améliore encore les effets précités.

L'ensemble formant mâchoires ou la mâchoire supérieure est également incliné(e) par rapport à l'axe longitudinal du corps cylindrique afin que l'élément longiligne adopte une inclinaison correspondante entre ledit ensemble et l'extrémité distale du corps.

Cette disposition évite également que l'élément longiligne ne dés-actionne le bloqueur anti-retour de l'ancillaire de mise en pré-tension selon l'invention qui sera décrit ci-après.

Dans une variante, les mâchoires (en particulier les mâchoires latérales gauche et droite) ou la mâchoire supérieure sont/est configurée(s) pour être mobile(s) et actionnable(s) par un organe de préhension pour le placement de l'élément longiligne entre lesdites mâchoires.

De préférence, l'ensemble formant mâchoires comprend un organe de verrouillage permettant de bloquer en position ouverte lesdites mâchoires. Encore de préférence, l'organe de verrouillage comprend deux ergots en vis-à-vis agencés dans ledit organe de préhension, notamment un lien, aptes à venir en butée contre le chariot afin de bloquer l'ensemble formant mâchoires en position ouverte.

Dans une variante, l'ancillaire comprend un dispositif de déverrouillage rapide pour découpler le chariot de l'arbre rotatif.

Cette disposition permet avantageusement de libérer automatiquement la tension sur l'élément longiligne, et forme ainsi une sécurité pour le chirurgien lors du réglage de l'élément longiligne.

Dans une variante, le dispositif d'embrayage et de débrayage comprend le dispositif de déverrouillage rapide permettant d'actionner le débrayage et l'embrayage du chariot avec l'arbre rotatif.

Dans une variante, le dispositif d'embrayage et de débrayage comprend une came montée sur un organe de rappel en liaison avec le dispositif de déverrouillage rapide permettant de faire passer le chariot de la position embrayée à la position débrayée et vice-versa.

Dans une sous-variante, le dispositif d'embrayage et de débrayage comprend un logement apte à recevoir l'arbre rotatif, ledit logement ayant une fenêtre apte à laisser passer une fourchette en liaison avec le chariot pour embrayer ou débrayer l'arbre rotatif sous l'effet de la came montée sur ledit organe de rappel.

Dans une variante, le corps comprend un ressort, notamment cylindrique, monté sur l'arbre rotatif et s'étendant longitudinalement entre l'extrémité proximale du corps et le chariot.

Le ressort prend appui au niveau de l'extrémité proximale du corps et contre le chariot, ce qui accélère la libération de la tension sur l'élément longiligne lorsque le dispositif de déverrouillage rapide est actionné pour découpler le chariot de l'arbre rotatif puisque le ressort pousse le chariot vers l'extrémité distale du corps.

Dans une variante, le chariot comporte une fente longitudinale pour recevoir l'élément longiligne, notamment deux portions dudit élément longiligne superposées.

Avantageusement, la tension est appliquée de façon homogène sur l'élément longiligne formant une boucle autour de la portion du corps vertébral et de la barre longitudinale.

Dans une sous-variante, la fente est disposée au-dessus de l'ensemble formant mâchoires.

Cette disposition facilite l'accès à l'ensemble formant mâchoires et ainsi la disposition de l'élément longiligne dans ledit ensemble.

Dans une variante, le dispositif de déverrouillage rapide comprend un levier qui est configuré lorsqu'il est actionné pour découpler le chariot de l'arbre rotatif.

Dans une variante, l'extrémité proximale du corps comprend un organe de couplage apte à être couplé avec une poignée amovible et configuré en sorte d'autoriser le pivotement de la poignée autour de l'axe longitudinal du corps.

De préférence, l'organe de couplage est également configuré pour bloquer dans une position déterminée la poignée autour de l'axe longitudinal du corps.

Avantageusement, le chirurgien peut ainsi modifier la position de la poignée en faisant pivoter cette dernière autour du corps selon l'axe longitudinal du corps. De préférence, la poignée amovible comprend une bague ayant sur son pourtour au moins une dent apte à coopérer avec un cran d'une bague crantée montée sur l'extrémité proximale du corps. Encore de préférence, l'organe de couplage comprend une bague de serrage venant serrer la bague comportant au moins une dent et la bague crantée afin de bloquer le couplage entre lesdites bagues. En particulier, l'extrémité proximale du corps comprend une portion filetée sur laquelle peut être vissée la bague de serrage.

La présente invention a pour objet, selon un deuxième aspect, un ensemble comprenant un ancillaire de mise en tension selon l'une quelconque des variantes de réalisation précédentes, et un ancillaire de mise en pré-tension comprenant un dispositif d'appui, ledit dispositif pour prendre appui sur l'implant est le dispositif d'appui dudit ancillaire de mise en pré-tension.

Dans une variante, l'ancillaire de mise en pré-tension de l'élément longiligne, notamment plat, pour la fixation de l'implant sur un élément osseux, notamment au moins une portion d'un corps vertébral, en entourant au moins partiellement ledit élément osseux, comprend une tige ayant des extrémités proximale et distale, l'extrémité distale étant munie d'un dispositif d'appui sur ledit implant, ladite tige s'étendant selon un axe longitudinal. Avantageusement, la tige comprend un dispositif de guidage et de blocage de l'élément longiligne selon l'axe longitudinal et ledit dispositif de guidage et de blocage comprend un bloqueur anti-retour autorisant le déplacement longitudinal de l'élément longiligne dans ledit dispositif de guidage et de blocage vers l'extrémité proximale de la tige et empêchant le déplacement longitudinal de l'élément longiligne vers l'extrémité distale de la tige.

Avantageusement, le bloqueur anti-retour empêche tout déplacement de l'élément longiligne vers l'extrémité distale de la tige lorsqu'aucune tension n'est exercée sur l'élément longiligne ce qui facilite le travail du chirurgien et lui permet de laisser en attente l'élément longiligne pour procéder à d'autres actes. Le chirurgien peut reprendre la mise en tension de l'élément longiligne sans avoir à exercer d'autre action préalable sur l'ancillaire de mise en pré-tension.

Avantageusement, l'élément longiligne est mis en tension selon une trajectoire parallèle à l'axe longitudinal de la tige entre l'extrémité distale de la tige et le bloqueur anti-retour.

Avantageusement, l'élément longiligne est mis en tension selon une trajectoire parallèle à l'axe longitudinal de la tige de l'ancillaire de mise en pré-tension et à l'axe longitudinal du corps de l'ancillaire de mise en tension, les axes longitudinaux du corps et de la tige étant parallèles.

De préférence, le bloqueur anti-retour est configuré pour recevoir au moins deux portions d'élément longiligne superposées, lesdites portions étant les prolongements respectivement des première et seconde extrémités libres de l'élément longiligne.

Le dispositif d'appui vient en appui sur l'implant qui peut comprendre un connecteur et/ou un moyen de solidarisation implantable de l'élément longiligne à une barre longiligne et/ou une barre longiligne.

Dans une variante, le bloqueur anti-retour comprend un organe de déverrouillage autorisant, lorsqu'il est actionné, le déplacement longitudinal de l'élément longiligne vers l'extrémité distale de la tige.

Avantageusement, l'organe de déverrouillage permet de relâcher automatiquement la tension exercée sur l'élément longiligne ce qui constitue une sécurité pour le chirurgien.

De préférence, le bloqueur anti-retour comprend une came montée sur un ressort de rappel et venant engager au moins une portion de l'élément longiligne, de préférence au moins deux portions superposées de l'élément longiligne, contre une surface d'appui, notamment supérieure, afin de bloquer le déplacement de l'élément longiligne vers l'extrémité distale de la tige.

De préférence, l'organe de déverrouillage est un levier qui agit sur la came et la désengage de l'élément longiligne.

Dans une variante, le bloqueur anti-retour comprend un passage longitudinal ayant une surface d'appui supérieure constituant une portion de guidage pour le déplacement longitudinal de l'élément longiligne.

Avantageusement, lorsque l'élément longiligne est plat, les frottements avec la surface d'appui sont amplifiés et améliorent le guidage et le blocage de l'élément longiligne contre ladite surface d'appui supérieure.

Dans une variante, le dispositif de guidage et de blocage comprend en outre un élément de blocage configuré pour bloquer l'élément longiligne contre la surface d'appui supérieur tout en empêchant le déplacement longitudinal de l'élément longiligne vers l'extrémité distale de la tige.

De préférence, l'élément de blocage comprend un organe de rappel dans sa position initiale correspondant à son engagement et donc au blocage de l'élément longiligne contre ladite surface d'appui. Lorsque l'élément longiligne est mis en tension entrainant son déplacement vers l'extrémité proximale de la tige, l'élément de blocage sous l'effet de l'élément longiligne, se désengage de ce dernier et donc de la surface d'appui supérieure.

De préférence, cette surface d'appui supérieure est inclinée par rapport à l'axe longitudinal de la tige pour éviter que l'élément de blocage, en particulier une came montée sur un ressort de rappel, reste en contact avec l'élément longiligne et se désengage dudit élément longiligne sous l'effet de l'élément longiligne lui-même selon l'angle d'application de la tension sur ce dernier.

A titre d'exemple, le ressort peut être cylindrique ou plat, et de préférence cylindrique.

En particulier, l'organe de rappel est monté entre l'organe de déverrouillage comprenant un levier d'actionnement et l'élément de blocage, de préférence autour d'une tige reliant ledit levier et l'élément de blocage.

En particulier, l'élément de blocage vient bloquer l'élément longiligne contre la surface d'appui, ainsi lorsque l'élément longiligne comprend au moins deux portions superposées (notamment dans le prolongement de chacune de ses première et seconde extrémités libres), une première portion est disposée entre la surface d'appui supérieure et la seconde portion tandis que la seconde portion est disposée entre la première portion et l'élément de blocage.

Dans une variante, l'élément de blocage est monté rotatif autour d'un axe transversal à la direction longitudinale de la tige.

De préférence, l'élément de blocage présente une portion de contact courbe avec l'élément longiligne afin d'éviter d'endommager par abrasion ce dernier.

Dans une variante, le dispositif de guidage et de blocage est configuré pour être traversé par deux portions superposées de l'élément longiligne.

Avantageusement, chacune des portions est dans le prolongement d'une des deux extrémités libres de l'élément longiligne de sorte que la tension exercée sur l'élément longiligne est appliquée uniformément sur l'ensemble de la boucle entourant au moins partiellement ledit corps vertébral ce qui évite tout risque de glissement dudit élément longiligne et donc tout risque d'érosion dudit corps vertébral.

De préférence, le dispositif de guidage et de blocage comprend une ouverture latérale facilitant l'introduction desdites portions superposées d'élément longiligne.

Dans une variante, l'organe de déverrouillage comprend un levier configuré pour actionner l'élément de blocage.

Avantageusement, le levier est en liaison avec l'élément de blocage et apte à faire pivoter ledit élément de blocage vers l'extrémité proximale de la tige afin d'autoriser le déplacement de l'élément longiligne (des deux portions superposées) vers ladite extrémité distale de la tige.

Dans une variante, l'extrémité distale de la tige comporte une tête qui comprend le dispositif d'appui, la tête est munie d'un logement configuré pour recevoir au moins une partie de l'implant, ce logement comprend une ouverture pour le passage et le guidage de l'élément longiligne.

De préférence, le logement est configuré pour recevoir un connecteur ou un moyen de solidarisation implantable de l'élément longiligne à une barre longitudinale.

De préférence, l'ouverture peut être un conduit débouchant sur une fenêtre ou une gorge.

Dans une variante, l'ouverture comprend une paroi de fond qui est incliné par rapport à l'axe longitudinal de la tige, pour guider l'élément longiligne vers le dispositif de guidage et de blocage.

Avantageusement, lors de l'intervention chirurgicale, l'implant, en particulier le connecteur ou le moyen de solidarisation implantable est ainsi dégagé et permet au chirurgien d'accéder à ce dernier facilement avec un tournevis par exemple pour terminer la fixation du l'élément longiligne sans qu'il ne soit gêné par les épineuses pour accéder à la zone d'implantation.

Dans une variante, la tête est inclinée par rapport à la l'axe longitudinal de la tige et forme une zone d'appui sur l'implant inclinée par rapport à un plan passant perpendiculairement à l'axe longitudinale.

On retrouve l'effet technique décrit précédemment.

Dans une variante, l'ouverture forme un conduit débouchant sur une fenêtre, ledit conduit ayant un orifice d'entrée et un orifice de sortie de l'élément longiligne distants d'une distance déterminée.

Avantageusement, lorsque l'élément longiligne sort du connecteur ou du moyen de solidarisation implantable, il est guidé par la fenêtre pour être mis en tension non pas directement en sorte de l'implant mais en sortie de la fenêtre afin de ne pas gêner la mise en oeuvre de l'organe de fixation de l'implant pour fixer l'élément longiligne audit implant.

Dans une variante, le conduit comprend des parois supérieure et inférieure, la paroi inférieure étant la paroi de fond inclinée.

Dans une variante, l'ouverture comprend un organe de couplage, notamment amovible, avec l'implant pour solidariser de manière amovible la tête avec l'implant.

De préférence, l'organe de couplage comprend des masselottes montées sur un organe de rappel, par exemple un ressort plat, apte à coopérer avec un organe de couplage correspondant sur l'implant, par exemple des ouvertures latérales traversantes.

Dans une variante, l'extrémité proximale de la tige est pourvue d'un organe de couplage avec un ancillaire de mise en tension.

L'extrémité proximale peut recevoir une poignée.

La présente invention sera mieux comprise à la lecture des exemples de réalisation décrits ci-après, cités à titre non limitatif et illustrés par les dessins décrits ci-après annexés au présent texte.

### Description détaillée des dessins

- La figure **1** est une représentation schématique et en perspective d'un connecteur implantable fixant un élément longiligne disposé autour d'une portion d'un corps vertébral et d'une barre longitudinale ;
- La figure **2** est une représentation schématique et en perspective partiellement éclatée d'une première variante d'un ancillaire de mise en tension selon l'invention ;
- La figure **3** est une représentation schématique et en perspective de l'ancillaire de mise en tension représenté à la figure **2** ;
- La figure **4** est une représentation vue de côté de l'ancillaire représenté aux figures **2** et **3** ;
- La figure **5** est une représentation vue de dessus de l'ancillaire représenté aux figures **2, 3** et **4** ;
- La figure **6** est une représentation éclatée du dispositif d'embrayage et de débrayage du chariot sur l'arbre rotatif de l'ancillaire représenté aux figures **2** à **5****;**
- La figure **7** est une représentation schématique et en perspective du dispositif d'embrayage et de débrayage du chariot sur l'arbre rotatif de l'ancillaire représenté aux figures **2** à **5** ;
- La figure **8** est une représentation schématique et en perspective d'un exemple d'ancillaire de mise en pré-tension selon l'invention ;
- La figure **9** est représentation schématique et en perspective d'un ensemble selon la présente invention comprenant l'ancillaire de mise en tension représentée aux figures **2** à **5** et l'ancillaire de mise en pré-tension représenté à la figure **8** en fonctionnement ;
- La figure **10** représente de façon schématique une variante de l'ensemble formant mâchoires du dispositif de fixation représenté sur les figures **2** et **3** ; et
- La figure **11** est une représentation éclatée de l'ensemble formant mâchoires représenté à la figure **10****.**

### Description détaillée des exemples de réalisation

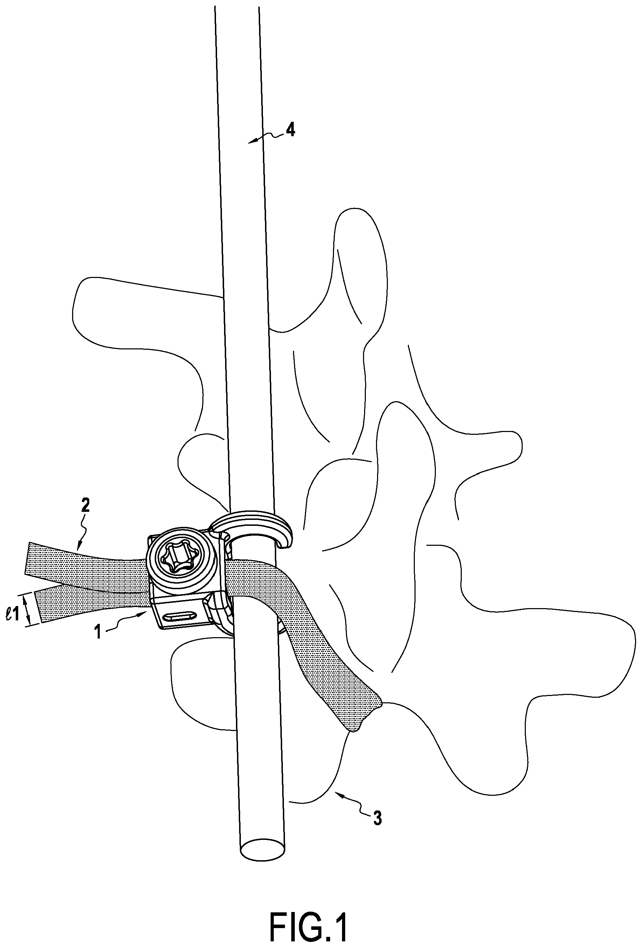

La figure **1** représente un connecteur implantable **1** (désigné dans le présent texte également en tant qu'implant) fixant un élément longiligne **2** de largeur **l1,** notamment plat, disposé autour d'une portion d'un corps vertébral **3** et d'une barre longitudinale **4.** Ledit élément longiligne **2** préalablement à sa fixation dans son état enroulé autour de la portion du corps vertébral **3** a été mis en tension à l'aide d'un ancillaire de mise en tension selon l'invention, éventuellement combiné avec un ancillaire de mise en pré-tension selon l'invention.

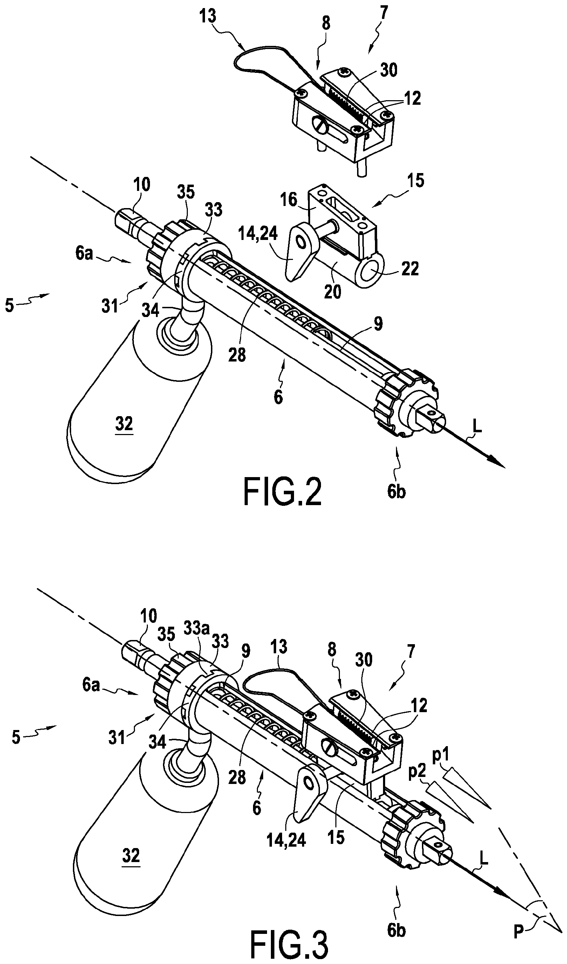

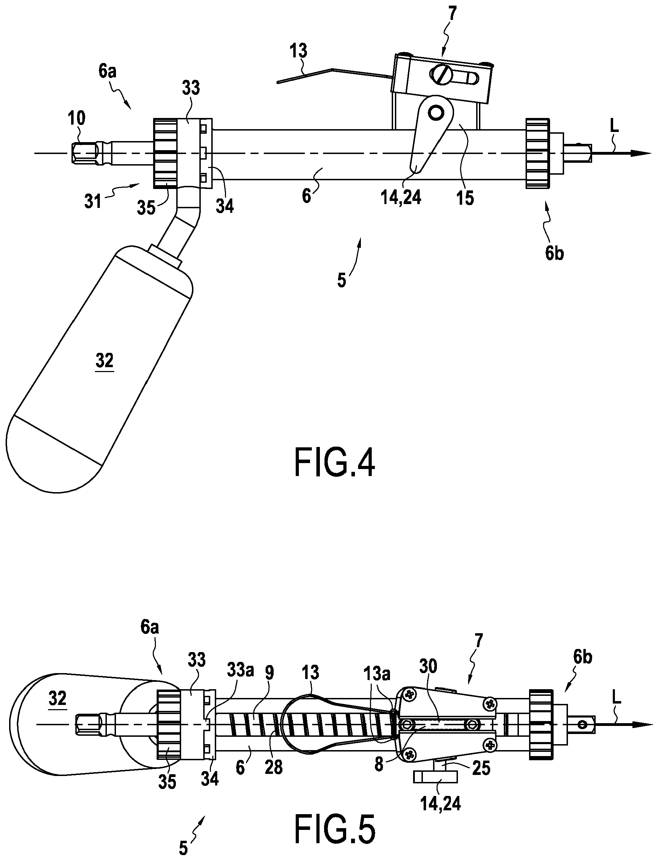

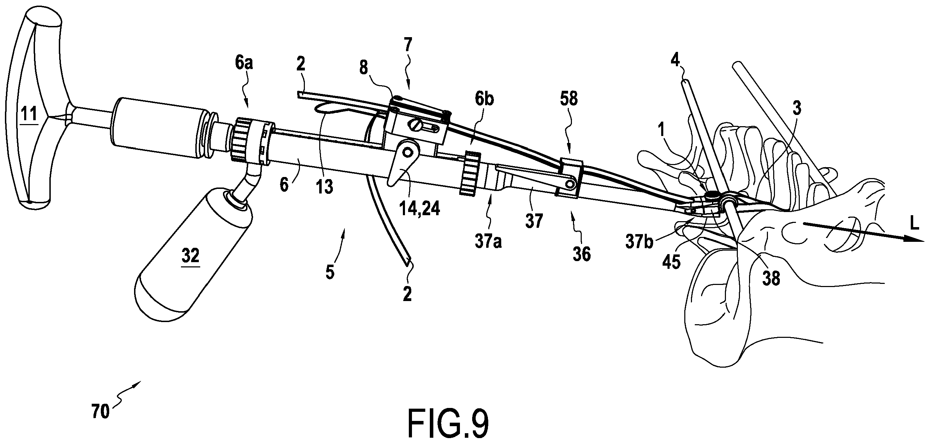

Les figures **2** à **5** représentent un premier exemple d'ancillaire de mise en tension **5** selon l'invention comprenant un corps **6** ayant des extrémités proximale **6a** et distale **6b** et s'étendant selon un axe longitudinal **L.** L'ancillaire de mise en tension **5** comprend en outre un chariot **7** mobile selon l'axe longitudinal **L** et un dispositif de fixation **8** à l'élément longiligne **2.** L'ancillaire **5** comprend également un arbre rotatif **9** autour de l'axe longitudinal **L** du corps **6,** cet arbre **9** est configuré pour coopérer avec le chariot **7** de sorte qu'une rotation de l'arbre **9** entraine le déplacement en translation du chariot **7** le long de l'axe longitudinal **L** et corrélativement l'éloignement du chariot **7** de l'extrémité distale **6b** du corps **6.** L'arbre **9** comporte une portion **10** s'étendant au-delà de l'extrémité proximale **6a** du corps **6** et configurée pour être couplée avec un dispositif d'actionnement dynamométrique, tel que le dispositif **11** représenté à la figure **9****.** Le dispositif de fixation **8** comporte un ensemble formant mâchoires **12,** s'étendant dans deux plans **p1,p2** inclinés l'un par rapport à l'autre et inclinés par rapport à l'axe longitudinal du corps **L.** Les mâchoires **12** sont configurées pour être mobiles et actionnables par un organe de préhension **13** pour le placement de l'élément longiligne **2** entre lesdites mâchoires **12.** Dans cet exemple précis, l'ensemble formant mâchoires **12** comprend un organe de verrouillage **13a** permettant de bloquer en position ouverte lesdites mâchoires **12.** L'organe de verrouillage **13a** comprend ainsi deux ergots en vis-à-vis agencés dans l'organe de préhension **13** et aptes à venir en butée contre le chariot **7** afin de bloquer l'ensemble formant mâchoires **12** en position ouverte.

L'ancillaire de mise en tension **5** comprend un dispositif de déverrouillage rapide **14** pour découpler le chariot **7** de l'arbre rotatif **9.** L'ancillaire **5** comprend un dispositif d'embrayage et de débrayage **15** permettant de faire passer le chariot **7** d'une position débrayée dans laquelle le chariot **7** est libre de glisser longitudinalement par rapport à l'axe longitudinal **l** de l'arbre rotatif **9** à une position embrayée dans laquelle le chariot **7** est couplé avec l'arbre rotatif **9,** ou inversement par l'intermédiaire du dispositif de déverrouillage rapide **14.** Le dispositif d'embrayage et de débrayage **15** comprend un support **16** logeant une came **17** en liaison avec une enclume **18** se projetant d'une fourchette **19** ayant une surface intérieure de contact **19a** partiellement annulaire comprenant un filetage correspondant à celui de l'arbre rotatif **9.** Le dispositif **15** comprend également une pièce en forme de tube creux **20** comprenant une fenêtre **21** apte à recevoir en translation selon l'axe **A** la fourchette **19.** Ladite pièce **20** comprend ainsi un logement **22** apte à recevoir une partie de l'arbre rotatif **9.** Le dispositif **15** comprend également deux organes de rappel **23,** notamment des ressorts sous forme de lames, disposés entre la face inférieure de l'enclume **18** et la face supérieure de la fourchette **19.** Le dispositif **15** comprend en outre un organe d'actionnement **24** faisant office de dispositif de déverrouillage rapide **14,** tel qu'un levier, monté rotatif selon un axe **T** transversal à l'axe longitudinal **L** du cylindre **6.** Les différentes pièces du dispositif **15** sont solidarisées notamment par l'intermédiaire des éléments de fixation **27.** L'organe d'actionnement **24** est ainsi configuré pour, lorsqu'il est actionné, découpler le chariot **7** de l'arbre rotatif **9.**

Par ailleurs, le corps **6** comprend un ressort **28** monté sur l'arbre rotatif **9** et s'étendant longitudinalement entre l'extrémité proximale **6a** du corps **6** et le chariot **7.** Le chariot **7** comporte une fente longitudinale **30** pour recevoir l'élément longiligne, notamment deux portions dudit élément longiligne superposés. De préférence, les mâchoires **12** sont dentelées. La fente **30** est disposée au-dessus de l'ensemble formant mâchoires **12.**

L'extrémité proximale **6b** du corps **6** comprend un organe de couplage **31** apte à être couplé avec une poignée amovible **32** et configuré en sorte d'autoriser le pivotement de la poignée **32** autour de l'axe longitudinal **L** du corps **6.** La poignée amovible **32** comprend une bague ayant sur son pourtour au moins une dent **33a** apte à coopérer avec un cran d'une bague crantée **34** montée sur l'extrémité proximale **6a** du corps **6**. Encore de préférence, l'organe de couplage **31** comprend une bague de serrage **35** venant serrer la bague **33** comportant au moins une dent **33a** et la bague crantée **34** afin de bloquer le couplage entre lesdites bagues **33,34.** En particulier, l'extrémité proximale **6a** du corps **6** comprend une portion filetée sur laquelle peut être vissée la bague de serrage **35.**

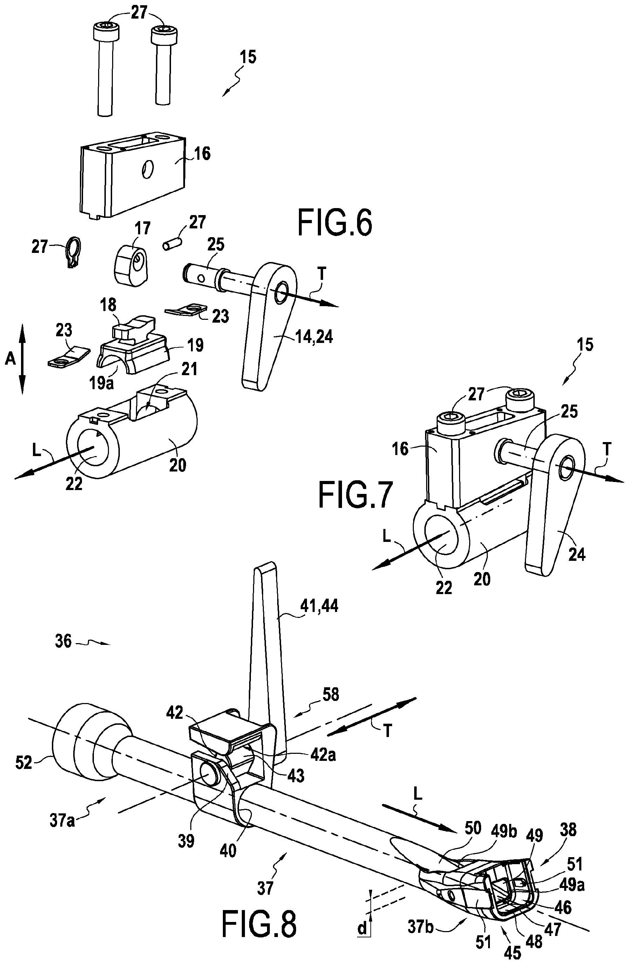

La figure **8** représente un ancillaire de mise en pré-tension **36** utilisé dans un ensemble **70** selon l'invention comprenant également l'ancillaire de mise en tension **5** et représenté en fonctionnement à la figure **9****.** L'ancillaire de mise en pré-tension **36,** représenté à la figure **8****,** comprend une tige **37** ayant des extrémités proximale **37a** et distale **37b,** l'extrémité distale **37b** étant munie d'un dispositif d'appui **38** sur un implant, dans cet exemple précis sur un connecteur implantable, tel que le connecteur **1** représenté à la figure **1****.** La tige **37** s'étend selon un axe longitudinal **L** et comprend un dispositif de guidage et de blocage **58** d'un élément longiligne, tel que l'élément longiligne **2,** selon l'axe longitudinal **L.** Le dispositif de guidage et de blocage **58** comprend un bloqueur anti-retour **39** autorisant le déplacement longitudinal selon l'axe longitudinal **L** de l'élément longiligne dans le dispositif de guidage et de blocage **58** vers l'extrémité proximale **37a** de la tige **37** et empêchant le déplacement longitudinal selon l'axe longitudinal **L** de l'élément longiligne vers l'extrémité distale **37b** de la tige **37.** Dans cet exemple précis, la tige **37** est configurée pour être reçue dans un logement **40** ménagé dans le dispositif de guidage et de blocage **58** pour la solidarisation de la tige **37** au dispositif de guidage et de blocage **58.** Le bloqueur anti-retour **39** comprend un organe de déverrouillage **41** autorisant, lorsqu'il est actionné, le déplacement longitudinal de l'élément longiligne vers l'extrémité distale **37b** de la tige **37.** Le bloqueur anti-retour **39** comprend un passage longitudinal **42** ayant une surface d'appui supérieure **42a** constituant une portion de guidage pour le déplacement longitudinal de l'élément longiligne. Le dispositif de guidage et de blocage **58** comprend en outre un élément de blocage **43** configuré pour bloquer l'élément longiligne contre la surface d'appui supérieur **42a** tout en empêchant le déplacement longitudinal de l'élément longiligne vers l'extrémité distale **37b** de la tige **37.** L'élément de blocage **43** est monté rotatif autour d'un axe **T** transversal à la direction longitudinale **L** de la tige **37.** L'organe de déverrouillage **41** comprend un levier **44** configuré pour actionner l'élément de blocage **43.** Le dispositif de guidage et de blocage **58** est configuré pour être traversé par deux portions superposées de l'élément longiligne, chacune desdites portions étant dans le prolongement d'une première ou seconde extrémité libre de l'élément longiligne. L'élément de blocage **43** est dans cet exemple précis une came montée sur un organe de rappel, notamment un ressort cylindrique.

L'extrémité distale **37b** de la tige **37** comporte une tête **45** qui comprend le dispositif d'appui **38,** la tête **45** est munie d'un logement **46** configuré pour recevoir au moins une partie de l'implant, notamment au moins partiellement le connecteur **1.** Ce logement **46** comprend une ouverture **47** pour le passage et le guidage de l'élément longiligne. L'ouverture **47** comprend une paroi de fond **48** inclinée par rapport à l'axe longitudinal **L** de la tige **37,** pour guider l'élément longiligne vers le dispositif de guidage et de blocage **58.** L'ouverture **47** forme un conduit **49** débouchant sur une fenêtre **50,** ledit conduit **49** ayant un orifice d'entrée **49a** et un orifice de sortie **49b** de l'élément longiligne distants d'une distance déterminée **d.** Le conduit **49** comprend des parois supérieur et inférieure, la paroi inférieure étant la paroi de fond **48** inclinée. L'ouverture **47** comprend un organe de couplage **51** avec l'implant, en particulier le connecteur **1,** pour solidariser de manière amovible la tête **45** avec l'implant, ici le connecteur **1.** L'extrémité proximale **37a** de la tige **37** est pourvue d'un organe de couplage **52** avec l'extrémité distale **6b** du corps **6** de l'ancillaire de mise en tension **5.**

A la figure **9****,** l'ancillaire de mise en tension **5** est illustré en fonctionnement couplé avec l'ancillaire de mise en pré-tension **36** pour des raisons de simplification. Néanmoins, à la lecture de la présente description, l'homme du métier comprend aisément que l'ancillaire de mise en tension **5** peut mettre en tension un élément longiligne indépendamment de l'ancillaire de mise en pré-tension **36** pour autant que l'ancillaire de mise en tension **5** comprenne un dispositif d'appui sur l'implant en particulier ménagé au niveau de l'extrémité distale **6b** du corps **6.** Par exemple, le dispositif d'appui **38** comprenant la tête **45** de l'ancillaire de mise en pré-tension **36** peut être ménagé au niveau ou dans le prolongement de l'extrémité distale **6b** du corps **6.** Dans l'ensemble **70** représenté en fonctionnement à la figure **9****,** l'ancillaire de mise en pré-tension **36** fait office de dispositif d'appui pour l'ancillaire de mise en tension **5.** Lors de la mise en tension de l'élément longiligne **2,** les première et seconde extrémités libres de l'élément longiligne en sortie du connecteur **1** et de la tête **45,** en particulier de l'orifice de sortie **49b** du conduit **49,** sont passées ensemble dans le dispositif de guidage et de blocage **58** puis dans le dispositif de fixation **8** du chariot **7.** Le chirurgien peut alors exercer une première traction manuelle pour tendre les portions superposées d'élément longiligne disposées dans les dispositifs **8** et **58** tout en ouvrant l'ensemble formant mâchoires **12** à l'aide de l'organe de préhension **13.** Le chirurgien peut également régler la position de la poignée **32** en la faisant pivoter autour de l'axe longitudinal **L** du corps **6** pour mieux accéder à la zone d'implantation. Le chirurgien accouple alors le dispositif dynamométrique **11** avec la portion **10** se projetant de l'arbre **9** et actionne le dispositif **11** ce qui provoque le déplacement du chariot **7** vers l'extrémité distale **6a** du corps **6** et corrélativement la mise en tension de l'élément longiligne **2.** On observe également le déplacement longitudinal de l'élément longiligne **2** dans le dispositif de guidage et de blocage **58.** Avantageusement, l'élément longiligne **2** se déplace selon les axes longitudinaux du corps **6** et de la tige **37** qui sont parallèles, notamment confondus. Le dispositif dynamométrique **11** est de préférence à déclenchement ce qui permet de ne pas excéder une force de serrage pré-déterminée. Si besoin, il est possible de relâcher immédiatement la tension par l'intermédiaire du dispositif de déverrouillage rapide **14** comprenant le levier **24** permettant ainsi de débrayer le chariot **7** de l'arbre rotatif **9.** Il est possible de ré-embrayer le chariot **7** sur l'arbre rotatif **9** en actionnant le dispositif de déverrouillage **14.** La tension appliquée sur l'élément longiligne **2** est relâchée automatiquement et ce de façon accélérée grâce au ressort **28.** Avantageusement, quand bien même la tension appliquée sur l'élément longiligne est relâchée au niveau du chariot **7,** l'élément longiligne **2** reste en tension par l'intermédiaire du dispositif de guidage et de blocage **58** comprenant un bloqueur anti-retour. Il est également possible, si besoin, de relâcher la tension au niveau du dispositif de guidage et de blocage **58** par l'intermédiaire de l'organe de déverrouillage **41** comprenant le levier **44.**

La fenêtre **39** permet de décaler d'environ la distance **d,** la sortie de l'élément longiligne **2** du connecteur **1.** Cette disposition permet de décaler l'application de la tension sur la fenêtre **50** et non de l'appliquer directement sur le connecteur **1** limitant ainsi les risques de découplage du connecteur **1** à la tête **45.**

En outre, l'inclinaison des plans **p1** et **p2** de l'ensemble formant mâchoires **12** par rapport à l'axe longitudinal **L** du corps **6** évite que l'élément longiligne **2** lorsqu'il est mis en tension ne désengage l'élément de blocage **43** de la surface d'appui supérieure **42a**.

Dans cet exemple précis, le dispositif d'appui **38** vient en appui sur le connecteur **1** et non sur la barre longitudinale **4.**

Lorsque l'élément longiligne est correctement mis en tension et le niveau vertébral corrigé, le chirurgien actionne le dispositif de fixation de l'élément longiligne à l'implant, dans cet exemple le connecteur **1.** Le chirurgien peut également avantageusement découpler l'ancillaire de mise en tension **5** de l'ancillaire **36** et régler la tension sur d'autres éléments longilignes sans actionner le dispositif de fixation de l'élément longiligne au connecteur afin de corriger plusieurs niveaux vertébraux si besoin.

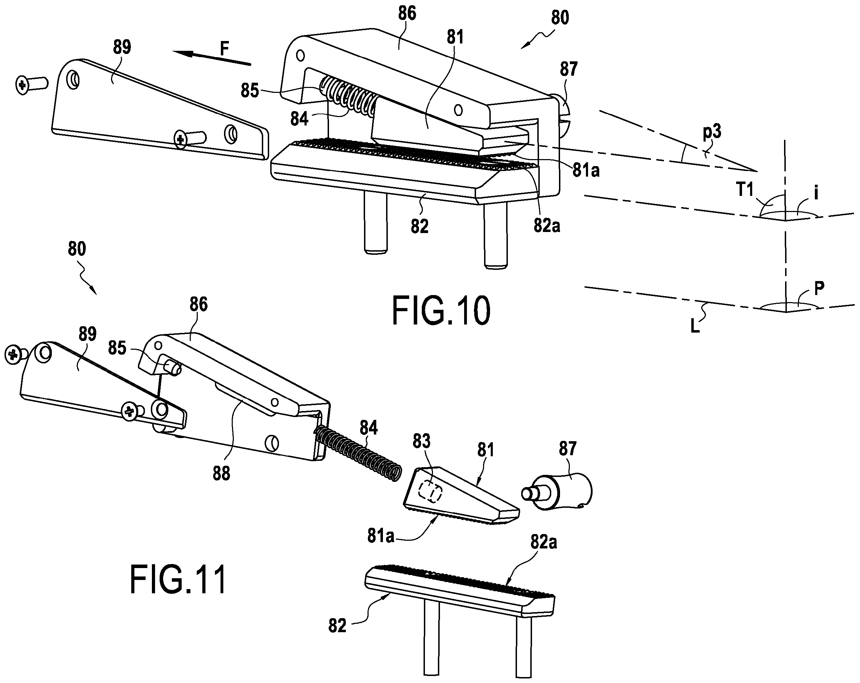

La figure **10** représente une variante de l'ensemble formant mâchoires **12** du dispositif de fixation **8** représenté sur les figures **2** et **3****.** L'ensemble formant mâchoires **80** représenté à la figure **10** est pivoté de 90° comparativement à l'ensemble formant mâchoires 12 de la figure **2****.**

Tel que représenté également à la figure **11****,** cet ensemble formant mâchoires **80** comprend une mâchoire supérieure **81** et une mâchoire inférieure **82,** la mâchoire supérieure **81** s'étendant dans un plan **p3** incliné par rapport à la mâchoire inférieure **82** et/ou par rapport à l'axe longitudinal **L** du corps cylindrique **6.** La mâchoire inférieure **82** est dans un plan **i** parallèle au plan longitudinal **P** du corps cylindrique **6.** La mâchoire supérieure **81** s'écarte de la mâchoire inférieure **82** (qui reste fixe, car solidarisée au dispositif d'embrayage et de débrayage), pour recevoir au moins une portion de l'élément longiligne, dans un plan **T1** transversal, en particulier sensiblement perpendiculaire, au plan longitudinal **P** du corps cylindrique **6.**

Plus particulièrement, la mâchoire supérieure **81** comporte un premier logement **83** recevant une première extrémité d'un ressort **84,** la seconde extrémité du ressort **84** coopère avec une projection **85** se projetant de la pièce principale **86** formant en partie l'ensemble formant mâchoires **80.** La mâchoire supérieure **81** comprend un second logement (non visible sur les figures) configuré pour recevoir une partie de l'organe de couplage **87.** La pièce principale **86** comporte également une lumière **88** dans laquelle l'organe de couplage **87** en liaison avec le second logement de la mâchoire supérieure **81** translate.

L'ensemble formant mâchoires **80** comprend également un cache **89** venant se fixer sur la pièce principale **86** diminuant ainsi la taille de l'ouverture latérale pour le passage de l'élément longiligne.

En fonctionnement, la mâchoire supérieure **81** est écartée de la mâchoire inférieure **82** en exerçant une traction selon la flèche **F** sur un organe de préhension non représenté, tel que l'organe de préhension **13** représenté sur les figures **2** et **3****,** en liaison avec la projection **85** afin de disposer l'élément longiligne **2** entre les deux mâchoires **81,82.** Lorsque la traction exercée pour comprimer le ressort **84** est relâchée, la mâchoire supérieure **81** revient dans sa position au repos et bloque l'élément longiligne **2** par pincement contre la mâchoire inférieure **82.**

Dans cet exemple précis, les mâchoires supérieure **81** et inférieure **82,** comprennent chacune une face ayant une portion de contact **81a,82a** venant en contact avec au moins une portion de l'élément longiligne 2. Lesdites portions de contact **81a,82a** sont moletées, en particulier comprennent un moletage de forme pyramidale. Encore de préférence, le moletage de forme pyramidale d'une portion de contact donnée **81a** est disposé en quinconce par rapport au moletage de la portion de contact opposée **82a** afin d'améliorer le pincement et donc le blocage de l'élément longiligne.

L'ensemble formant mâchoires **80** permet avantageusement que l'élément longiligne **2** (au moins deux portions de ce dernier superposées) pincé entre lesdites mâchoires **81** et **82,** soit disposé dans un plan parallèle au plan longitudinal **P** du corps cylindrique **6,** ce qui lui évite de vriller.

## Revendications

1. Ancillaire de mise en tension (5) d'un élément longiligne (2) pour la fixation d'un implant (1) sur un élément osseux, notamment au moins une portion d'un corps vertébral (3), en entourant au moins partiellement ledit élément osseux, ledit ancillaire (5) comprenant un corps (6) ayant des extrémités proximale (6a) et distale (6b), l'extrémité distale (6a) étant munie d'un dispositif pour prendre appui sur l'implant, ledit corps (6) s'étendant selon un axe longitudinal (L), l'ancillaire (5) comprend en outre un chariot (7) mobile selon l'axe longitudinal (L) et un dispositif de fixation (8) à l'élément longiligne (2), **caractérisé en ce que** l'ancillaire (5) comprend un arbre rotatif (9) autour de l'axe longitudinal (L) du corps (6), cet arbre (9) est configuré pour coopérer avec le chariot (7) de sorte qu'une rotation de l'arbre (9) entraine le déplacement en translation du chariot (7) le long de l'axe longitudinal (L) et corrélativement l'éloignement du chariot (7) de l'extrémité distale (6b) du corps (6) et la mise en tension de l'élément longiligne (2), et **en ce que** l'ancillaire (5) comprend un dispositif d'embrayage et de débrayage du chariot (7) de l'arbre rotatif (9), le chariot (7) ayant une position débrayée dans laquelle le chariot (7) est libre de glisser longitudinalement par rapport à l'axe longitudinal (I) de l'arbre rotatif (9) et une position embrayée dans laquelle le chariot (7) est couplé avec l'arbre rotatif (9).

2. Ancillaire (5) selon la revendication **1, caractérisé en ce que** l'arbre (9) comporte une portion (10) s'étendant au-delà de l'extrémité proximale (6a) du corps (6) configurée pour être couplée avec un dispositif d'actionnement dynamométrique (11).

3. Ancillaire (5) selon l'une ou l'autre des revendications **1** et **2, caractérisé en ce que** le dispositif de fixation (8) comporte un ensemble formant mâchoires (12), s'étendant dans deux plans (p1,p2) inclinés l'un par rapport à l'autre et inclinés par rapport à l'axe longitudinal du corps (L).

4. Ancillaire (5) selon la revendication **3, caractérisé en ce que** les mâchoires (12) sont configurées pour être mobiles et actionnables par un organe de préhension (13) pour le placement de l'élément longiligne (2) entre lesdites mâchoires (12).

5. Ancillaire (5) selon l'une quelconque des revendications **1** à **4, caractérisé en ce que** l'ancillaire (5) comprend un dispositif de déverrouillage rapide (14) pour découpler le chariot (7) de l'arbre rotatif (9).

6. Ancillaire (5) selon la revendication **5, caractérisé en ce que** le dispositif de déverrouillage rapide (14) comprend un levier (24) qui est configuré lorsqu'il est actionné pour découpler le chariot (7) de l'arbre rotatif (9).

7. Ancillaire (5) selon l'une quelconque des revendications **1** à **6, caractérisé en ce que** le corps (6) comprend un ressort (28) monté sur l'arbre rotatif (9) et s'étendant longitudinalement entre l'extrémité proximale (6a) du corps (6) et le chariot (7).

8. Ancillaire (5) selon l'une quelconque des revendications **1** à **7, caractérisé en ce que** le chariot (7) comporte une fente longitudinale (30) pour recevoir l'élément longiligne (2), notamment deux portions dudit élément longiligne (2) superposés.

9. Ancillaire (5) selon la revendication **8, caractérisé en ce que** la fente (30) est disposée au-dessus de l'ensemble formant mâchoires (12).

10. Ancillaire (5) selon l'une quelconque des revendications **1** à **9, caractérisé en ce que** l'extrémité proximale (6a) du corps (6) comprend un organe de couplage (31) apte à être couplé avec une poignée amovible (32) et configuré en sorte d'autoriser le pivotement de la poignée (32) autour de l'axe longitudinal (L) du corps (6).

11. Ensemble (70) comprenant un ancillaire de mise en tension (5) selon l'une quelconque des revendications **1** à **10, caractérisé en ce qu'**il comprend un ancillaire de mise en pré-tension (36) comprenant un dispositif d'appui, et **en ce que** ledit dispositif pour prendre appui sur l'implant est le dispositif d'appui dudit ancillaire de mise en pré-tension (36).

12. Ensemble (70) selon la revendication **11, caractérisé en ce que** l'ancillaire de mise en pré-tension (36) comprend une tige (37) ayant des extrémités proximale et distale (37a,37b), l'extrémité distale (37b) étant munie d'un dispositif d'appui (38) sur ledit implant, ladite tige s'étendant selon un axe longitudinal, et comprend un dispositif de guidage et de blocage (58) de l'élément longiligne (2) selon l'axe longitudinal (L), ledit dispositif de guidage et de blocage (58) comprend un bloqueur anti-retour (39) autorisant le déplacement longitudinal de l'élément longiligne dans ledit dispositif de guidage et de blocage (58) vers l'extrémité proximale (37a) de la tige (37) et empêchant le déplacement longitudinal de l'élément longiligne vers l'extrémité distale (37b) de la tige (37).

13. Ensemble (70) selon l'une quelconque des revendications **11** et **12, caractérisé en ce que** le bloqueur anti-retour (39) comprend un organe de déverrouillage (41) autorisant, lorsqu'il est actionné, le déplacement longitudinal de l'élément longiligne vers l'extrémité distale (37b) de la tige (37).

14. Ensemble (70) selon l'une quelconque des revendications **11** à **13, caractérisé en ce que** le bloqueur anti-retour (39) comprend un passage longitudinal (42) ayant une surface d'appui supérieure (42a) constituant une portion de guidage pour le déplacement longitudinal de l'élément longiligne.

15. Ensemble (70) selon l'une des revendications **11** à **14, caractérisé en ce que** le dispositif de guidage et de blocage (58) comprend en outre un élément de blocage (43) configuré pour bloquer l'élément longiligne contre la surface d'appui supérieur (42a) tout en empêchant le déplacement longitudinal de l'élément longiligne vers l'extrémité distale (37b) de la tige (37).

## Patentansprüche

1. Zubehör zum Spannen (5) eines länglichen Elements (2) zur Befestigung eines Implantats (1) an einem Knochenelement, insbesondere mindestens einem Abschnitt eines Wirbelkörpers (3), indem es das Knochenelement mindestens teilweise umgibt, wobei das Zubehör (5) einen Körper (6) umfasst, der proximale (6a) und distale Enden (6b) aufweist, wobei das distale Ende (6a) mit einer Vorrichtung zum Aufliegen auf dem Implantat versehen ist, wobei sich der Körper (6) entlang einer Längsachse (L) erstreckt, wobei das Zubehör (5) ferner einen Schlitten (7), der entlang der Längsachse (L) bewegbar ist, und eine Vorrichtung zur Befestigung (8) an dem länglichen Element (2) umfasst, **dadurch gekennzeichnet, dass** das Zubehör (5) eine Drehwelle (9) um die Längsachse (L) des Körpers (6) umfasst, wobei diese Welle (9) dazu ausgelegt ist, dass sie derart mit dem Schlitten (7) zusammenwirkt, dass die Drehung der Welle (9) die Translationsbewegung des Schlittens (7) entlang der Längsachse (L) und entsprechend die Entfernung des Schlittens (7) von dem distalen Ende (6b) des Körpers (6) und das Spannen des länglichen Elements (2) bewirkt, und dadurch, dass das Zubehör (5) eine Vorrichtung zum Ein- und Ausrücken des Schlittens (7) in und aus der Drehwelle (9) umfasst, wobei der Schlitten (7) eine ausgerückte Position aufweist, in der der Schlitten (7) ungehindert in Längsrichtung relativ zur Längsachse (1) der Drehwelle (9) gleiten kann, und eine eingerückte Position, in der der Schlitten (7) mit der Drehwelle (9) gekoppelt ist.

2. Zubehör (5) gemäß Anspruch 1, **dadurch gekennzeichnet, dass** die Welle (9) einen Abschnitt (10) umfasst, der sich über das proximale Ende (6a) des Körpers (6) hinaus erstreckt, der eingerichtet ist, um mit einer Drehmomentbetätigungsvorrichtung (11) gekoppelt zu werden.

3. Zubehör (5) gemäß einem der Ansprüche 1 und 2, **dadurch gekennzeichnet, dass** die Befestigungsvorrichtung (8) eine Backenanordnung (12) umfasst, die sich in zwei Ebenen (p1, p2) erstreckt, die bezogen aufeinander geneigt sind und bezogen auf die Längsachse des Körpers (L) geneigt sind.

4. Zubehör (5) gemäß Anspruch 3, **dadurch gekennzeichnet, dass** die Backen (12) derart eingerichtet sind, dass sie durch ein Greiforgan (13) bewegbar und betätigbar sind, um das längliche Element (2) zwischen den beiden Backen (12) anzuordnen.

5. Zubehör (5) gemäß einem der Ansprüche 1 bis 4, **dadurch gekennzeichnet, dass** das Zubehör (5) eine Schnelllösevorrichtung (14) zum Entkoppeln des Schlittens (7) von der Drehachse (9) umfasst.

6. Zubehör (5) gemäß Anspruch 5, **dadurch gekennzeichnet, dass** die Schnelllösevorrichtung (14) einen Hebel (24) umfasst, der eingerichtet ist, um bei Betätigung den Schlitten (7) von der Drehwelle (9) zu entkoppeln.

7. Zubehör (5) gemäß einem der Ansprüche 1 bis 6, **dadurch gekennzeichnet, dass** der Körper (6) eine Feder (28) umfasst, die an der Drehwelle (9) angebracht ist und sich in Längsrichtung zwischen dem proximalen Ende (6a) des Körpers (6) und dem Schlitten (7) erstreckt.

8. Zubehör (5) gemäß einem der Ansprüche 1 bis 7, **dadurch gekennzeichnet, dass** der Schlitten (7) einen Längsschlitz (30) umfasst, um das längliche Element (2), insbesondere zwei übereinandergelegte Abschnitte des länglichen Elements (2), aufzunehmen.

9. Zubehör (5) gemäß Anspruch 8, **dadurch gekennzeichnet, dass** der Schlitz (30) oberhalb der Backenanordnung (12) angeordnet ist.

10. Zubehör (5) gemäß einem der Ansprüche 1 bis 9, **dadurch gekennzeichnet, dass** das proximale Ende (6a) des Körpers (6) ein Kopplungsorgan (31) umfasst, das mit einem abnehmbaren Griff (32) gekoppelt werden kann und derart eingerichtet ist, dass es das Schwenken des Griffs (32) um die Längsachse (L) des Körpers (6) zulässt.

11. Anordnung (70), umfassend ein Zubehör zum Spannen (5) gemäß einem der Ansprüche 1 bis 10, **dadurch gekennzeichnet, dass** sie ein Zubehör zum Vorspannen (36) umfasst, das eine Auflagevorrichtung umfasst, und dadurch, dass die Vorrichtung zum Aufliegen auf dem Implantat die Auflagevorrichtung des Zubehörs zum Vorspannen (36) ist.

12. Anordnung (70) gemäß Anspruch 11, **dadurch gekennzeichnet, dass** das Zubehör zum Vorspannen (36) eine Stange (37) umfasst, die proximale und distale Enden (37a, 37b) umfasst, wobei das distale Ende (37b) mit einer Vorrichtung zum Aufliegen (38) auf dem Implantat versehen ist, wobei sich die Stange entlang einer Längsachse erstreckt und eine Vorrichtung zum Führen und zum Blockieren (58) des länglichen Elements (2) entlang der Längsachse (L) umfasst, wobei die Vorrichtung zum Führen und zum Blockieren (58) eine Rücklaufsperre (39) umfasst, die die Bewegung in Längsrichtung des länglichen Elements in der Vorrichtung zum Führen und zum Blockieren (58) zum proximalen Ende (37a) der Stange (37) zulässt und die Bewegung in Längsrichtung des länglichen Elements zum distalen Ende (37b) der Stange (37) verhindert.

13. Anordnung (70) gemäß einem der Ansprüche 11 und 12, **dadurch gekennzeichnet, dass** die Rücklaufsperre (39) eine Lösevorrichtung (41) umfasst, die bei Betätigung die Bewegung in Längsrichtung des länglichen Elements zum distalen Ende (37b) der Stange (37) zulässt.

14. Anordnung (70) gemäß einem der Ansprüche 11 bis 13, **dadurch gekennzeichnet, dass** die Rücklaufsperre (39) einen Längskanal (42) umfasst, der eine obere Auflagefläche (42a) aufweist, die einen Führungsabschnitt für die Bewegung in Längsrichtung des länglichen Elements bildet.

15. Anordnung (70) gemäß einem der Ansprüche 11 bis 14, **dadurch gekennzeichnet, dass** die Vorrichtung zum Führen und zum Blockieren (58) ferner ein Blockierelement (43) umfasst, das eingerichtet ist, um das längliche Element gegen die obere Auflagefläche (42a) zu blockieren, während es die Bewegung in Längsrichtung des länglichen Elements zum distale Ende (37b) der Stange (37) verhindert.

## Claims

1. A tensioning ancillary instrument (5) for tensioning an elongate element (2) to fasten an implant (1) on a portion of bone, in particular at least a portion of a vertebral body (3), by surrounding said portion of bone at least in part, said instrument (5) comprising a body (6) having proximal and distal ends (6a, 6b), the distal end (6a) being provided with a device for bearing against the implant, said body (6) extending along a longitudinal axis (L), the instrument (5) further comprising a carriage (7) that is movable along the longitudinal axis (L), and a fastener device (8) for fastening to the elongate element (2), the instrument (5) being **characterized in that** the instrument (5) includes a rotary shaft (9) rotatable about the longitudinal axis (L) of the body (6), the shaft (9) being configured to co-operate with the carriage (7) in such a manner that rotation of the shaft (9) drives movement of the carriage (7) in translation along the longitudinal axis (L) and correspondingly moves the carriage (7) away from the distal end (6b) of the body (6) and tensions the elongate element (2), and **in that** the instrument (5) includes a clutching and declutching device for clutching and declutching the carriage (7) and the rotary shaft (9), the carriage (7) having a declutched position in which the carriage (7) is free to slide longitudinally relative to the longitudinal axis (ℓ) of the rotary shaft (9), and a clutched position in which the carriage (7) is coupled with the rotary shaft (9).

2. An instrument (5) according to claim 1, **characterized in that** the shaft (9) includes a portion (10) extending beyond the proximal end (6a) of the body (6) and configured to be coupled with a torque actuator device (11).

3. An instrument (5) according to claim 1 or claim 2, **characterized in that** the fastener device (8) comprises a jaw-forming assembly (12) extending in two planes (p1, p2) that slope relative to each other and that slope relative to the longitudinal axis (L) of the body.

4. An instrument (5) according to claim 3, **characterized in that** the jaws (12) are configured to be movable and actuatable by a grip member (13) in order to place the elongate element (2) between said jaws (12).

5. An instrument (5) according to any one of claims 1 to 4, **characterized in that** the instrument (5) includes a rapid unlocking device (14) for decoupling the carriage (7) from the rotary shaft (9).

6. An instrument (5) according to claim 5, **characterized in that** the rapid unlocking device (14) includes a lever (24) that is configured, when actuated, to decouple the carriage (7) from the rotary shaft (9).

7. An instrument (5) according to any one of claims 1 to 6, **characterized in that** the body (6) includes a spring (28) mounted on the rotary shaft (9) and extending longitudinally between a proximal end (6a) of the body (6) and the carriage (7).

8. An instrument (5) according to any one of claims 1 to 7, **characterized in that** the carriage (7) includes a longitudinal slot (30) for receiving the elongate element (2), in particular two superposed portions of said elongate element (2).

9. An instrument (5) according to claim 8, **characterized in that** the slot (30) is arranged above the jaw-forming assembly (12).

10. An instrument (5) according to any one of claims 1 to 9, **characterized in that** the proximal end (6a) of the body (6) includes a coupling member (31) suitable for being coupled with a removable handle (32) and configured so as to allow the handle (32) to pivot about the longitudinal axis (L) of the body (6).

11. An assembly (70) comprising a tensioning instrument (5) according to any one of claims 1 to 10, **characterized in that** it includes a pre-tensioning ancillary instrument (36) including a bearing device, and **in that** said device for bearing against the implant is the bearing device of said pre-tensioning instrument (36).

12. An assembly (70) according to claim 11, **characterized in that** the pre-tensioning ancillary instrument (36) includes a rod (37) having proximal and distal ends (37a,37b), the distal end (37b) being provided with a bearing device (38) for bearing against said implant, said rod extending along a longitudinal axis and includes a guide and a blocking device (58) for guiding and blocking the elongate element (2) along the longitudinal (L) axis, and said guide and blocking device (58) includes an anti-reverse blocker (39) allowing the elongate element to move in said guide and blocking device(58) longitudinally towards the proximal end (37a) of the rod (37), and preventing the elongate element moving longitudinally towards the distal end (37b) of the rod (37).

13. An assembly (70) according to any one of claims 11 and 12, **characterized in that** the anti-reverse blocker (39) includes an unlocking member (41) that, when actuated, allows the elongate element to move longitudinally towards the distal end (37b) of the rod (37).

14. An assembly (70) according to any one of claims 11 to 13, **characterized in that** the anti-reverse blocker (39) has a longitudinal passage (42) with a top bearing surface (42a) constituting a guide portion for guiding the longitudinal movement of the elongate element.

15. An assembly (70) according to any one of claims 11 to 14, **characterized in that** the guiding and blocking device (58) further comprises a blocking element (43) configured to block the elongate element against the top bearing surface (42a), while preventing the elongate element from moving longitudinally towards the distal end (37b) of the rod (37).
